# EUROPEAN PATENT APPLICATION

(11) **EP 2 011 491 A1**
(43) Date of publication of application: **07.01.2009**
(21) Application number: 07301208.0
(22) Date of filing: 05.07.2007
(51) Int. Cl.: A61K 31/185, A61K 31/197, A61K 45/06, A61P 25/08

(54) **Anticonvulsive pharmaceutical compositions**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Catherine, Alain

(57) **Abstract**

The use of a substance selected from the group consisting of taurine, a taurine precursor, a taurine metabolite, a taurine derivative, a taurine analog and a substance required for the taurine biosynthesis, for manufacturing a pharmaceutical composition for preventing or inhibiting the undesirable side-effects caused to a human or an animal organism by an active ingredient that induces a high level of extracellular GABA or increases GABA receptor activation.

## Description

### FIELD OF THE INVENTION

The present invention relates to the field of human or animal therapy using anticonvulsive active ingredients that induce a high level of extracellular GABA or activate GABA receptors. These active anticonvulsive ingredients are used for preventing various neurological disorders including epilepsy.

More particularly, this invention is aimed at reducing the undesirable effects caused to a human or an animal organism by these anticonvulsive active ingredients, including damages to the retina.

### BACKGROUND OF THE INVENTION

GABA is important to several neurological disorders, including Parkinson's disease, Huntington's chorea, Alzheimer's disease, and epilepsy, a central nervous system disease characterized by recurring convulsive seizures.

A deficiency of GABA in the brain has been implicated as one cause for convulsions. (Karlsson, A.; Funnum, F.; Malthe-Sorrensen, D.; Storm-Mathisen, J. Biochem Pharmacol 1974, 22, 3053-3061.) In an effort to raise the concentration of GABA in the brain, both direct injection and oral administration of GABA have been studied. It was shown that injection of GABA into the brain has an anticonvulsant effect, but it is obviously not a practical method. Taking GABA orally, however, is not effective because GABA cannot cross the blood-brain barrier, a membrane protecting the CNS from xenobiotics in the blood.

To correct the deficiency of brain GABA and therefore stop convulsions, an important approach is to use an inhibitor of GABA-aminotransferase (GABA-AT) that is able to cross blood-brain barrier. (Nanavati, S. M.; Silverman, R. B. J. Med. Chem. 1989, 32, 2413-2421.). Inhibition of this enzyme increases the concentration of GABA in the brain, which has therapeutic applications in epilepsy as well as other neurological disorders. One of the most effective in vivo time-dependent inhibitors of GABA-AT is 4-amino-5-hexenoic acid, which is also termed gamma-vinyl GABA or vigabatrin, an anticonvulsant drug marketed almost all over the world.

Other ways for correcting the deficiency of brain GABA relies on increasing GABA transmission by blocking GABA transporters like tiagabine, acting at GABA receptors (topiramate or felbamate) or activating the glutamate decarboxylase to increase GABA synthesis (pyridoxal phosphate, valproate, gabapentin). All strategies have in common to increase the concentration of GABA in the brain or directly activate its receptors and thus stimulate GABA therapeutic effects in various neurological disorders including epilepsy.

Regarding more particularly epilepsy, even though existing antiepileptic drugs can render 80% of newly diagnosed patients seizure free, a significant number of patients have chronic intractable epilepsy causing disability with considerable socioeconomic implications.

Anti-epileptic drugs are available for treating epilepsies, but these agents have a number of shortcomings. For instance, the agents are often poorly soluble in aqueous and biological fluids or are extremely hygroscopic. Of even greater importance is that patients often become refractory to a drug over time. In addition, many anti-epileptic agents cause unwanted side effects, neurotoxicities, and drug interactions. Even while being treated with one or a combination of the anti-epileptic drugs currently in clinical use, 30% of epileptic patients still experience seizures. As more anti-epileptic drugs are developed, the clinician will have expanded pharmaceutical options when designing an effective treatment protocol for each patient.

Vigabatrin, which was developed as an inhibitor of gamma-aminobutyric acid transaminase, was one of the most promising novel anticonvulsant active ingredients. However, vigabatrin, like some other compounds that increase GABA levels, can induce highly severe undesirable effects, such as an irreversible constriction of the visual field. The constriction of the visual field induced by vigabatrin is asymptomatic when it is restricted to the nasal quadrant, until it extends to more central areas. Furthermore, visual defects induced by vigabatrin are not limited to the constriction of the visual field but also includes dysfunction of central vision with a reduction of visual acuity, a loss of color discrimination and of contrast sensitivity. An arrest of a therapeutical treatment with vigabatrin allows a stabilization of the visual loss but very rarely induces any recovery.

However, because epileptic seizures are always very handicapping and may be lethal, active ingredients that increase the GABA levels, including vigabatrin, are still prescribed. Similarly, visual side effects were also described with many other anti-epileptic molecules blocking the GABA transaminase (valproate), increasing GABA levels by blocking GABA transporter (tiagabine), stimulating GABA synthesis and/or release (gabapentin, valproate, levetiracetam), or increasing GABA receptor activation (topiramate, felbamate, benzodiazepines like diazepam, clonazepam and clobazam or barbiturates like primidone and phenobarbitone).

There is thus a need in the art for substances or methods that would allow avoiding side effects that are caused to the human or animal organisms which are treated by anticonvulsive active ingredients that increase GABA levels or activate GABA receptors.

There is thus a need in the art for improved anticonvulsive, including anti-epileptic, pharmaceutical treatments based on anticonvulsive active ingredients, which would be endowed with reduced or no undesirable effects.

### SUMMARY OF THE INVENTION

The present invention provides for the use of taurine, a taurine precursor, a taurine metabolite, a taurine derivative, a taurine analog and a substance required for the taurine biosynthesis, for manufacturing a pharmaceutical composition for preventing or inhibiting the undesirable side-effects caused to a human or an animal organism by an active ingredient that induces an increase of extracellular GABA or GABA transmission.

Preferred active ingredients that induce a high level of extracellular GABA, for which side effects are prevented or inhibited, are selected from the group consisting of GABA-aminotransferase inhibitors, GABA transport inhibitors, GABA receptor activators and glutamate decarboxylase activators.

The most preferred active ingredient, for which side effects are prevented or inhibited, consists of 4-amino-5-hexenoic acid, also termed vigabatrin. The active ingredient is either the racemic mixture or the active isomer.

This invention also provides for pharmaceutical compositions comprising, as the active ingredients, a combination of :
(i) a first active ingredient that induces a high level of extracellular GABA or increases GABA transmission; and
(ii) a second active ingredient selected from the group consisting of taurine, a taurine precursor, a taurine metabolite, a taurine derivative, a taurine analog and a substance required for the taurine biosynthesis,
and one or more pharmaceutically acceptable excipients.

The present invention also pertains to a method for treating convulsive disorders, including epilepsy, comprising a step of administering, to a patient in need thereof, a combination of :
(i) a first active ingredient that induces a high level of extracellular GABA or increases GABA transmission; and
(ii) a second active ingredient selected from the group consisting of taurine, a taurine precursor, a taurine metabolite, a taurine derivative, a taurine analog and a substance important for taurine biosynthesis like vitamin B6.

### BRIEF DESCRIPTION OF THE FIGURES

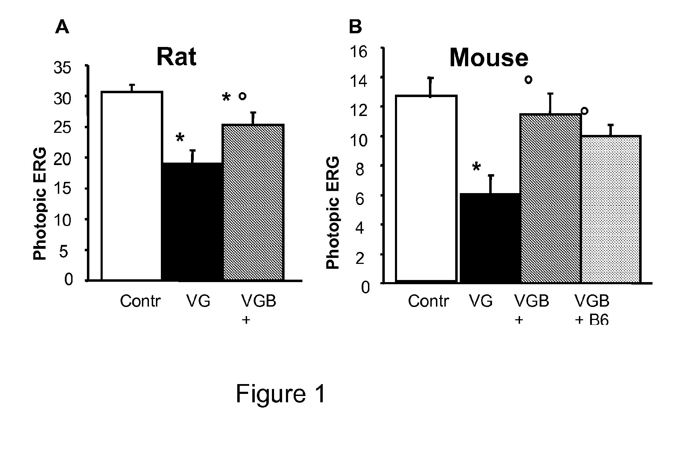

Figure 1 illustrates the neuroprotection by taurine or vitamin B6 against the side effects caused by the extracellular GABA-inducer vigabatrin.

When rats (Figure 1A) and mice (Figure 1 B) were treated with vigabatrin, a supplementation with taurine or vitamin B6 prevented or partially suppressed the decrease in amplitude of the photopic electroretinogram (ERG) observed under vigabatrin alone. The differences in the photopic ERG amplitudes were statistically significant between the control and vigabatrin treated animals (VGB: * p<0.05). By contrast, in mice, the differences between control animals and animals treated with vigabatrin plus taurine or vigabatrin plus vitamin B6 were no longer significant. In rats, the difference remained significant with the control group (VGB+ taurine: * p<0.05). However, in both mice and rats, animals receiving vigabatrin plus taurine had smaller decrease in photopic ERG amplitude than animals treated with vigabatrin alone; the differences were statistically significant (VGB+ taurine, ° p<0.05).. Similarly, mice receiving vitamin B6 supplementation in addition to vigabatrin had smaller decrease in photopic ERG than with vigabatrin alone and the difference was statistically significant (VGB+ B6, ° p<0.05).

### DETAILED DESCRIPTION OF THE INVENTION

Surprisingly, it has been found according to the invention that the progressive and irreversible loss of visual acuity of patients treated with the extracellular GABA inducer vigabatrin is associated with a high decrease in circulating taurine concentration.

It has also been surprisingly found herein that a supplementation of vigabatrin-treated individuals with taurine or with a substance required for the taurine biosynthesis inhibits the vigabatrin-induced undesirable side effects, and inhibits particularly the vigabatrin-induced retinal lesions.

These inventor's findings were all the more surprising that it was previously shown in the art that vigabatrin induced no change in the levels of glutamate, aspartate, glycine or taurine, whereas it induced a four-fold increase in GABA levels.

It has thus been found according to the invention that the deleterious side effects that are caused by anticonvulsive active ingredients acting as extracellular GABA-inducers might be prevented or blocked by the administration of any one of the substances that increase the amount of circulating taurine within a human or an animal organism.

As intended herein, the substances that increase the amount of circulating taurine within a human or an animal organism encompass, in addition to taurine *per se,* also taurine precursors, taurine metabolites, taurine derivatives, taurine analogs and substances required for the taurine biosynthesis.

As intended herein, taurine precursors, taurine metabolites, taurine derivatives, taurine analogs and substances required for the taurine biosynthesis may be collectively termed "taurine-like" substances.

Thus, an object of the present invention consists of the use of a substance selected from the group consisting of taurine, a taurine precursor, a taurine metabolite, a taurine derivative, a taurine analog and a substance required for the taurine biosynthesis, for manufacturing a pharmaceutical composition for preventing or inhibiting the undesirable side-effects caused to a human or an animal organism by an active ingredient that induces a high level of extracellular GABA.

As used herein, taurine consists of the 2-aminoethanesulfonic acid.

As used herein, taurine precursors encompass substances that, when they are administered to a human or an animal, can be transformed, directly or indirectly, into taurine.

Preferred taurine precursors are selected from the group consisting of cysteine, cystathionine, homocysteine, S-adenosylhomocysteine, serine, N-acetyl-cysteine, glutathione, N-formylmethionine, S-adenosylmethionine, betaine and methionine.

As used herein, taurine metabolites encompass substances that are produced *in vivo* by transformation of taurine.

Preferred taurine metabolites are selected from the group consisting of hypotaurine, thiotaurine, taurocholate.

As used herein, taurine derivatives encompass substances that are structurally close to taurine but possess at least one structural difference, such as one or more chemical changes, e.g. at least one replacement of an atom or a chemical group found in taurine by a distinct atom or a distinct chemical group.

Preferred taurine derivatives are selected from different entities including the group consisting of acetylhomotaurinate, and piperidino-, benzamido-, phthalimido- or phenylsuccinylimido taurine derivatives. Such taurine derivatives are described notably by Kontro et al. (1983, Prog Clin Biol Res, Vol. 125 : 211-220) and by Andersen et al. (2006, Journal of pharmaceutical Sciences, Vol. 73(n°1) : 106-108). Derivatives include for instance taurolidine (4,4'-methylene-bis(tetrahydro-2H-1,2,4-thiadiazine-1,1-dioxide or taurolin), taurultam and taurinamide, chlorohydrate-N-isopropylamide-2-(1-phenylethyl)aminoethanesulfonic acid.

As used herein, taurine analogs encompass substances that are chemically distinct from taurine but which exert the same biological activity, e.g. an inhibition of the retinal lesions induced by the extracellular GABA inducer vigabatrin.

Preferred taurine analogs are selected from the group consisting of (+/)piperidine-3-sulfonic acid (PSA), 2-aminoethylphosphonic acid (AEP), (+/-)2-acetylaminocyclohexane sulfonic acid (ATAHS), 2-aminobenzenesulfonate (ANSA), hypotaurine,. ± trans-2-aminocyclopentanesulfonic acid (TAPS) 8-tétrahydroquinoléine sulfonic acid (THQS), N-2-hydroxyethylpiperazine-N'-2-ethane sulphonic acid (HEPES), beta-alanine, glycine, guanidinoethylsulfate (GES), 3-acétamido-1-propanesulfonic acid (acamprosate).

As used herein, the substances required for taurine biosynthesis encompass all substances that are involved in the *in vivo* taurine biosynthesis including enzymes and enzyme cofactors, thus including cysteine dioxygenase (EC 1.13.11), sulfinoalanine decarboxylase (EC 4.1.1.29) and cofactors thereof.

Preferred substances required for taurine biosynthesis are selected from the group consisting of vitamin B6 (or pyridoxal-5'-phosphate), vitamin B12 (cobalamin), folic acid, riboflavin, pyridoxine, niacin, thiamine (thiamine pyrophosphate) and pantothenic acid.

As used herein, active ingredients that induce a high level of extracellular GABA or increases GABA receptor activation encompass GABA-aminotransferase inhibitors, GABA transporter inhibitors, Glutamate decarboxylase activators and GABA receptor agonists or modulators.

GABA-aminotransferase, may also be termed GABA-transaminase or 4-aminobutyrate transaminase (EC 2.6.1.19). Glutamate decarboxylase is classified as EC 4.1.1.15.

As intended herein, GABA-aminotransferase inhibitors, the side effects of which are inhibited or blocked by taurine, encompass is 4-amino-5-hexenoic acid (vigabatrin), valproate, (1R,3S,4S)-3-amino-4-fluorocyclopentane-1-carboxylic acid, (1 R,4S)-4-amino-2-cyclopentene-1-carboxylic acid, (1S,4R)-4-amino-2-cyclopentene-1-carboxylic acid, (4R)-4-amino-1-cyclopentene-1-carboxylic acid, (4S)-4-amino-1-cyclopentene-1-carboxylic acid, (S)-4-amino-4,5-dihydro-2-thiophenecarboxylic acid, 1H-tetrazole-5-(alpha-vinyl-propanamine), 2,4-Diaminobutanoate, 2-Oxoadipic acid, 2-Oxoglutarate, 2-Thiouracil, 3-Chloro-4-aminobutanoate, 3-Mercaptopropionic acid, 3-Methyl-2-benzothiazolone hydrazone hydrochloride, 3-Phenyl-4-aminobutanoate, 4-ethynyl-4-aminobutanoate, 5-Diazouracil, 5-Fluorouracil, Aminooxyacetate, beta-Alanine, Cycloserine and D-Cycloserine.

As intended herein; glutamate decarboxylase activators, the side effects of which can be inhibited or blocked by taurine, encompass 2-Oxoglutarate, 3-Mercaptopropionic acid, Aminooxyacetic acid and Glutarate.

The GABA transporter inhibitor may consist of tiagabine.
The GABA receptors agonists and modulators : may be selected from the group consisting of topiramate, felbamate, tramadol, Oxcarbazepine, Carbamazepine, eszopiclone, zopiclone, baclofen, gamma-Hydroxybutyric acid, imidazopyridines like zaleplon, zolpidem, zopiclone phenytoin, propofol, phenytoin, benzodiazepines and barbiturates.

Benzodiazepines may be selected from the group consisting of clobazam, Alprazolam (Xanax®), Bromazepam (Lexomil®), Diazepam (Valium®), Lorazepam (Ativan®), Clonazepam (Klonopin®), Temazepam (Restoril®), Oxazepam (Serax®), Flunitrazepam (Rohypnol®), Triazolam (Halcion®), Chlordiazepoxide (Librium®), Flurazepam (Dalmane®), Estazolam (ProSom®), and Nitrazepam (Mogadon®).

Barbiturates may be selected from the group consisting of primidone and phenobarbitone, pentobarbital, midazolam, phenytoin, secobarbital and amobarbital butabarbital barbital, phenobarbital, butalbital, cyclobarbital, allobarbital, methylphenobarbital, and vinylbital.

The present invention also pertains to pharmaceutical compositions comprising taurine, a taurine precursor, a taurine metabolite, a taurine derivative, a taurine analog and a substance required for the taurine biosynthesis and wherein the said pharmaceutical compositions are aimed at preventing or inhibiting the undesirable side-effects caused to a human or an animal organism by an active ingredient that induces a high level of extracellular GABA or increases GABA transmission.

This invention also relates to therapeutic methods for preventing or inhibiting the undesirable side-effects caused to a human or an animal organism by an active ingredient that induces a high level of extracellular GABA or increases GABA transmission, wherein the said therapeutic methods comprise a step of administering, to a human or an animal organism in need thereof, an effective amount of a substance selected from the group consisting of taurine, a taurine precursor, a taurine metabolite, a taurine derivative, a taurine analog and a substance required for the taurine biosynthesis.

These compositions and methods are described in more detail hereunder.

### Pharmaceutical compositions and methods.

Generally, a pharmaceutical composition according to the invention comprises the combination of :
(i) a first active ingredient that induces a high level of extracellular GABA or increases GABA transmission; and
(ii) a second active ingredient selected from the group consisting of taurine, a taurine precursor, a taurine metabolite, a taurine derivative, a taurine analog and a substance required for the taurine biosynthesis,
and further also one or more pharmaceutically acceptable excipients.

Thus, the said second active ingredient is selected from the group consisting of taurine and "taurine-like" substances.

In a pharmaceutical composition according to the invention, a preferred active ingredient that induces a high level of extracellular GABA, i.e. the first active ingredient, consists of 4-amino-5-hexenoic acid (vigabatrin), the racemic mixture or the active isomer.

The pharmaceutical compositions according to the invention are aimed at preventing various neurological disorders including primarily convulsive disorders. Convulsive disorders encompass epilepsy, tuberous sclerosis, as well as the convulsive disorders affecting patients undergoing a drug addiction, including a drug addiction to heroin or cocaine, ethanol.

Thus, a pharmaceutical composition according to the invention consists primarily of an anti-convulsive pharmaceutical composition.

The first and the second active ingredients are comprised in an anticonvulsive pharmaceutical composition in a "therapeutically effective amount", that is in an amount sufficient for the combination of active ingredients to exert the expected anticonvulsive effect while inducing no deleterious side effect, or side effects which are reduced as compared with the deleterious side effects induced by pharmaceutical compositions comprising the first active ingredient without taurine nor a taurine-like substance.

Generally speaking, a "therapeutically effective amount", or "effective amount", or "therapeutically effective", as used herein, refers to that amount which provides a therapeutic effect for a given condition and administration regimen. This is a predetermined quantity of active material calculated to produce a desired therapeutic effect in association with the required additive and diluent; i.e., a carrier, or administration vehicle. Further, it is intended to mean an amount sufficient to reduce and most preferably prevent a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluents; i.e., carrier, or additive.

The present invention also deals with methods for preventing or inhibiting the undesirable side-effects caused to a human or an animal organism by an active ingredient that induces a high level of extracellular GABA or increases GABA transmission, wherein the said methods comprise a step of administering, to a human or an animal organism in need thereof, an effective amount of taurine or of a taurine-like substance.

The present invention also concerns methods for preventing or treating convulsive disorders comprising a step of administering, to a human or an animal organism in need thereof, a combination of :
(i) a first active ingredient that induces a high level of extracellular GABA or increases GABA transmission; and
(ii) a second active ingredient selected from the group consisting of taurine, a taurine precursor, a taurine metabolite, a taurine derivative, a taurine analog and a substance required for the taurine biosynthesis,

Typically, the first active ingredient consists of 4-amino-5-hexenoic acid (vigabatrin).

In an advantageous embodiment, the second active ingredient is selected form the group consisting of taurine and vitamin B6.

The individuals in need of such treatments encompass those, either adult or child patients, which are susceptible to convulsive disorders, especially epilepsy.

In certain embodiments, the said method comprises a step of administering to a patient in need thereof a pharmaceutical composition that is described in the present specification.

In a pharmaceutical composition according to the invention, the amount of the first active ingredient, i.e. the active ingredient that induces a high level of extracellular GABA, is the amount of the said active ingredient that is conventionally used for treating patients affected with convulsive disorders. Typically, for vigabatrin, the said pharmaceutical composition is adapted so that the dosage form used allows the administration of an amount of vigabatrin ranging between 0.5 grams and 5 grams per day, preferably between 1 gram and 3 grams for a human adult patient having a mean weight of 80 kilos. Typically, a dosage form will contain half the daily dose, for the purpose of performing two takes per day. Lower amounts of vigabatrin may be used, especially when the active ingredient is not under the racemic form but instead under the form of its active isomer, which lower amounts are typically half the amount of the racemic form which would have been conventionally used.

In a pharmaceutical composition according to the invention, the amount of the second active ingredient, i.e. taurine or a taurine-like substance, is adapted so that the said pharmaceutical composition is adapted so that the dosage form used allows the administration of an amount of taurine or of the taurine-like substance ranging from 10 mg to 10 grams per day for a human adult patient having a mean weight of 80 kilos.

Indeed, in a pharmaceutical composition or in a pharmaceutical method according to the invention, the active ingredient(s) is (are) used in combination with one or more pharmaceutically or physiologically acceptable excipients.

Generally, a pharmaceutical composition according to the invention, irrespective of whether the said composition (i) comprises only one or more substances selected from taurine and taurine-like substances or (ii) comprises a combination of a first active ingredient that induces a high level of extracellular GABA and a second active ingredient selected from taurine and taurine-like substances, comprises the one or more active ingredients in an amount ranging from 0.1 % to 99.9% by weight, and usually from 1% to 90% by weight, based on the total weight of the said pharmaceutical composition.

Generally, a pharmaceutical composition according to the invention comprises an amount of excipient(s) that ranges from 0.1 % to 99.9% by weight, and usually from 10% to 99% by weight, based on the total weight of the said pharmaceutical composition.

By "physiologically acceptable excipient or carrier" is meant solid or liquid filler, diluents or substance which may be safely used in systemic or topical administration. Depending on the particular route of administration, a variety of pharmaceutically acceptable carriers well known in the art include solid or liquid fillers, diluents, hydrotropes, surface active agents, and encapsulating substances.

Pharmaceutically acceptable carriers for systemic administration that may be incorporated in the composition of the invention include sugar, starches, cellulose, vegetable oils, buffers, polyols and alginic acid. Specific pharmaceutically acceptable carriers are described in the following documents, all incorporated herein by reference: U.S. Pat. No. 4,401,663, Buckwalter et al. issued August 30, 1983; European Patent Application No. 089710, LaHann et al. published Sept. 28, 1983; and European Patent Application No. 0068592, Buckwalter et al. published Jan. 5, 1983. Preferred carriers for parenteral administration include propylene glycol, pyrrolidone, ethyl oleate, aqueous ethanol, and combinations thereof.

Representative carriers include acacia, agar, alginates, hydroxyalkylcellulose, hydroxypropyl methylcellulose, carboxymethylcellulose, carboxymethylcellulose sodium, carrageenan, powdered cellulose, guar gum, cholesterol, gelatin, gum agar, gum arabic, gum karaya, gum ghatti, locust bean gum, octoxynol 9, oleyl alcohol, pectin, poly(acrylic acid) and its homologs, polyethylene glycol, polyvinyl alcohol, polyacrylamide, sodium lauryl sulfate, poly(ethylene oxide), polyvinylpyrrolidone, glycol monostearate, propylene glycol monostearate, xanthan gum, tragacanth, sorbitan esters, stearyl alcohol, starch and its modifications. Suitable ranges vary from about 0.5% to about 1%.

For formulating a pharmaceutical composition according to the invention, the one skilled in the art will advantageously refer to the last edition of the European pharmacopoeia or of the United States pharmacopoeia.

Preferably, the one skilled in the art will refer to the fifth edition "2005" of the European Pharmacopoeia, or also to the edition USP 28-NF23 of the United States Pharmacopoeia.

The weight amount of the combination of active ingredients that is contained in each dose of the pharmaceutical composition of the invention will depend on the molecular weight of said therapeutically active compound as well as on the weight amount that is effective in inhibiting or blocking the convulsive disorder. Effective amounts of extracellular GABA inducers, and specifically of vigabatrin, that are needed for preventing or treating convulsive disorders are well known from the one skilled in the art.

For determining the appropriate amount of taurine or of the taurine-like substance(s) to be combined with the extracellular GABA inducer, e.g. vigabatrin, in a dose of a pharmaceutical composition of the invention, the one skilled in the art may advantageously refer to the effective amounts that are already known or determined in the art for the taurine or taurine-like substance(s) that is (are) comprised therein.

The present invention is further illustrated by the examples below.

### EXAMPLES

### A. Materials and Methods

### Vigabatrin treatment

As described previously (Duboc et al., 2004), Wistar rats Rj Wi IOPS Han and Balb c mice were purchased from Janvier Breeding Center (Le Genest St Isle, France) at 7 weeks. VGB was dissolved in 0.9 % NaCl at a concentration of 125 mg/ml. It was daily injected in intraperitonealy at a final dose of 200mg/kg in rats and 150mg/kg in mice. Vitamin B6 (active form) was administered at a dose of 1 mM/kg (solution 154,46 mg/ml) whereas taurine was provided in the drinking water at 0,1 mol/l.

ERG were recorded as described previously after the last VGB injection (Duboc et al., 2004). Anesthesia was induced by an intramuscular injection of a solution containing ketamine (100 mg/kg) and xylazine (10 mg/kg). After 10 min adaptation to a 2.5 cdm⁻² light intensity, ten recordings were obtained with flash intensity of 10 cdsm⁻² at an interstimulus interval 30s and subsequently averaged. These photopic ERG measurements were performed after 59 days in rats and 29 days in mice.

After 65 days of injection, rats were anesthetized for blood sampling. Anesthesia was induced as described above. Under anesthesia an indwelling catheter was placed in the carotid artery. This method allowed obtaining a single, large, good quality blood sample. A 1 mL blood sampling was taken for each rat in a heparinized blood sampler, immediately centrifuged and frozen at -80°C. Automated amino acid ion chromatography (Pasteur Cerba, France) was used for the quantitative determination of total and free amino acids in the samples.

### Example 1 : taurine deficiency induced by the extracelular GABA inducer Vigabatrin

In Table 1, the amino acid concentrations measured on blood samples for both vigabatrin-treated and control albino animals are presented. It indicates that concentration differences ranging from 7 to 32 % were not statistically significant for most amino acids thréonine, sérine, valine, citrulline, alanine, glycine, proline, glutamine, isoleucine, leucine, tyrosine, phenylalanine, arginine, histidine, lysine, ornithyne. As previously reported the glutamic acid was greatly reduced by 56% in the blood sample and this difference was statistically significant. However, the greatest change was observed for taurine which was decreased by 67% in vigabatrin-treated animals from 373.4 to 122.2 µM. This difference was statistically significant (p<0.05). A significant difference (22%) was also observed for methionine. These observations indicated that taurine and methionine concentrations are significantly reduced by the vigabatrin treatment.

### Example 2 : prevention and treatment of the undesirable side effects caused by the extracellular GABA inducer Vigabatrin by taurine and a taurine-like substance

To investigate whether taurine supplementation could prevent the vigabatrin-induced retinal toxicity, animals were treated with vigabatrin alone or vigabatrin plus a taurine supplementation. Vigabatrin alone induced a decrease in the photopic electroretinogram (ERG) measurement as previously described. By contrast, in mice, the difference was no longer statistically significant between controls and vigabatrin plus taurine supplementation or vigabatrin plus vitamin B6 supplementation (Fig. 1 B). In rats, although the photopic ERG amplitude decreased less than in vigabatrin alone, the difference remained statistically significant with the group of control animals (Fig1A). However, in all species, the difference between vigabatrin and vigabatrin plus taurine supplementation was statistically significant in treated animals (Fig.1). Similarly, the supplementation with vitamin B6 also reduced the decrease in photopic ERG amplitude and the difference between the mouse group of vigabatrin alone and the group receiving vigabatrin plus vitamin B6 supplementation was statistically significant (Fig.1B). These results are consistent with the hypothesis that vigabatrin-induced decrease in taurine concentration could produce the retinal toxicity of vigabatrin.

**Table 1: Blood concentrations for amino acids in albino rats treated with vigabatrin (VGB) or not treated (control). Measures are provided in µM (n=5, s.e.m.).**

| | **Control** | **VGB** |
|---|---|---|
| Taurine | 373,4 ± 46,7 | 122,2 ± 26,6 * |
| Thréonine | 194,2 ± 30,8 | 130,6 ± 7,0 |
| Sérine | 203,8 ± 28,7 | 169,4 ± 16,1 |
| Acide glutamique | 217,4 ± 56,6 | 94,8 ± 15,6 * |
| Valine | 167,4 ± 14,8 | 146 ±8,9 |
| Citrulline | 54 ±9,7 | 45,6 ± 4,2 |
| Alanine | 303,8 ± 32,3 | 270,4 ± 29,5 |
| Glycine | 190,6 ± 30,1 | 142,8 ± 17,6 |
| Proline | 155 ± 21,0 | 110,6 ± 20,1 |
| Glutamine | 375,6 ± 40,7 | 349 ± 13,4 |
| Methionine | 58 ± 4,7 | 45 ± 1,4 * |
| Isoleucine | 79,8 ± 8,0 | 68,2 ± 4,5 |
| Leucine | 152,6 ± 17,6 | 123,2 ± 11,5 |
| Tyrosine | 63,2 ± 6,8 | 51,6 ± 4,5 |
| Phenylalanine | 54,2 ± 6, 9 | 42,8 ± 1,0 |
| Arginine | 130 ± 39,0 | 111,8 ± 15,1 |
| Histidine | 75,8 ± 5,4 | 63,2 ± 2,9 |
| Lysine | 307,2 ± 23,8 | 260,4 ± 32,3 |
| Ornithine | 78,6 ± 33,0 | 57,6 ±10,6 |

### Reference

Duboc A, Hanoteau N, Simonutti M, Rudolf G, Nehlig A, Sahel JA, Picaud S (2004) Vigabatrin, the GABA-transaminase inhibitor, damages cone photoreceptors in rats. Ann Neurol 55:695-705.

## Claims

1. The use of a substance selected from the group consisting of taurine, a taurine precursor, a taurine metabolite, a taurine derivative, a taurine analog and a substance required for the taurine biosynthesis, for manufacturing a pharmaceutical composition for preventing or inhibiting the undesirable side-effects caused to a human or an animal organism by an active ingredient that induces a high level of extracellular GABA or increases GABA transmission.

2. The use according to claim 1, wherein the taurine precursor is selected from the group consisting of cysteine, cystathionine, homocysteine, S-adenosylhomocysteine, serine, N-acetyl-cysteine, glutathione, N-formylmethionine, S-adenosylmethionine, betaine and methionine.

3. The use according to claim 1, wherein the taurine metabolite is selected from the group consisting of hypotaurine, thiotaurine, taurocholate.

4. The use according to claim 1, wherein the taurine derivative is selected from the group consisting of of acetylhomotaurinate, and piperidino-, benzamido-, phthalimido- or phenylsuccinylimido taurine derivatives taurolidine, taurultam and taurinamide, chlorohydrate-N-isopropylamide-2-(1-phenylethyl)aminoethanesulfonic acid.

5. The use according to claim 1, wherein the taurine analog is selected from the group consisting of (+/-)piperidine-3-sulfonic acid (PSA), 2-aminoethylphosphonic acid (AEP), (+/-)2-acetylaminocyclohexane sulfonic acid (ATAHS), 2-aminobenzenesulfonate (ANSA), hypotaurine,. ± trans-2-aminocyclopentanesulfonic acid (TAPS) 8-tétrahydroquinoléine sulfonic acid (THQS), N-2-hydroxyethylpiperazine-N'-2-ethane sulphonic acid (HEPES), beta-alanine, glycine, guanidinoethylsulfate (GES), 3-acétamido-1-propanesulfonic acid (acamprosate)

6. The use according to claim 1, wherein the substance required for the taurine biosynthesis is selected from the group consisting of vitamin B6, vitamin B12, folic acid, riboflavin, pyridoxine, niacin, thiamine, and pantothenic acid

7. The use according to any one of claims 1 to 6, wherein the active ingredient that induces a high level of extracellular GABA or increases GABA receptor activation is selected from the group consisting of a GABA-aminotransferase inhibitor, a GABA transporter blocker, a Glutamate decarboxylase activator and a GABA receptor agonist or modulator.

8. The use according to claim 7, wherein the GABA-aminotransferase inhibitor consists of 4-amino-5-hexenoic acid (vigabatrin), its racemic mixture or the active isomer, valproate, (1R,3S,4S)-3-amino-4-fluorocyclopentane-1-carboxylic acid, (1 R,4S)-4-amino-2-cyclopentene-1-carboxylic acid, (1S,4R)-4-amino-2-cyclopentene-1-carboxylic acid, (4R)-4-amino-1-cyclopentene-1-carboxylic acid, (4S)-4-amino-1-cyclopentene-1-carboxylic acid, (S)-4-amino-4,5-dihydro-2-thiophenecarboxylic acid, 1H-tetrazole-5-(alpha-vinyl-propanamine), 2,4-Diaminobutanoate, 2-Oxoadipic acid, 2-Oxoglutarate, 2-Thiouracil, 3-Chloro-4-aminobutanoate, 3-Mercaptopropionic acid, 3-Methyl-2-benzothiazolone hydrazone hydrochloride, 3-Phenyl-4-aminobutanoate, 4-ethynyl-4-aminobutanoate, 5-Diazouracil, 5-Fluorouracil, Aminooxyacetate, beta-Alanine, Cycloserine and D-Cycloserine.

9. The use according to claim 7 wherein the GABA transporter blocker consists of tiagabine.

10. The use according to claim 7, wherein the GABA receptors agonists or modulators are selected from the group consisting of, topiramate, felbamate, tramadol , Oxcarbazepine, Carbamazepine, eszopiclone, zopiclone, baclofen, gamma-Hydroxybutyric acid, imidazopyridines like zaleplon, zolpidem, zopiclone phenytoin, propofol, phenytoin, benzodiazepines and barbiturates.

11. A pharmaceutical composition comprising, as the active ingredients, a combination of :
(i) a first active ingredient that induces a high level of extracellular GABA or increases GABA transmission; and
(ii) a second active ingredient selected from the group consisting of taurine, a taurine precursor, a taurine metabolite, a taurine derivative, a taurine analog and a substance required for the taurine biosynthesis,
and one or more pharmaceutically acceptable excipients.

12. The pharmaceutical composition according to claim 11, wherein the first active ingredient that induces a high level of extracellular GABA consists of 4-amino-5-hexenoic acid, its racemic mixture or the active isomer.

13. The pharmaceutical composition according to claim 11, wherein the second active ingredient consists of taurine.
